# EUROPÄISCHE PATENTSCHRIFT

(11) **EP 0 891 948 B1**
(45) Veröffentlichungstag und Bekanntmachung des Hinweises auf die Patenterteilung: **09.04.2003**
(21) Anmeldenummer: 98112987.7
(22) Anmeldetag: 13.07.1998
(51) Int. Cl.: C01B 37/00, C01F 7/34, C01F 7/02, C09C 1/40, A61K 9/52, A61K 47/02, C12N 11/14, B01J 21/04

(54) **Aluminiumoxid enthaltender Feststoff mit grosser Oberfläche**
Aluminium oxide containing solid of high specific surface
Solide contenant de l'oxyde d'aluminium à grande surface spécifique

(30) Priorität: 14.07.1997 DE 19730126
(43) Veröffentlichungstag der Anmeldung: 20.01.1999
(73) Patentinhaber: BASF AKTIENGESELLSCHAFT, 67056 Ludwigshafen (DE)
(72) Erfinder: Müller, Ulrich, Dr., 67435 Neustadt (DE); Ruetz, Roger, 68163 Mannheim (DE)
(74) Vertreter: Isenbruck, Günter, Dr.

(56) Entgegenhaltungen:
- EP-A- 0 814 059
- WO-A-96/39357
- BAGSHAW STEPHEN A. ET AL.: "Mesoporous alumina molecular sieves" ANGEWANDTE CHEMIE. INTERNATIONAL EDITION., Bd. 35, Nr. 10, 1996, Seiten 1102-1105, XP002083325 WEINHEIM DE

## Beschreibung

Die Erfindung betrifft einen Aluminiumoxid enthaltenden Feststoff mit großer spezifischer Oberfläche, ein Verfahren zur Herstellung desselben, sowie die Verwendung von Membranlipiden als Strukturbildner.

Die Herstellung von Aluminiumoxid enthaltenden Feststoffen ist an sich bekannt. Eine Übersicht der momentan technisch ausgeübten Verfahren wird in "Ullmann's Encycl. Tech. Chem.", 5. Aufl. 1997, Seiten 561 - 562, und "Winnacker, Küchler", 4. Aufl., Hanser-Verl. 1983 Bd. 3, Seiten 2 - 41, beschrieben.

Ein derart hergestelltes Aluminiumoxid hat eine spezifische Oberfläche im Bereich von 100 bis 400 m²/g bei Porendurchmessern von 2 bis 5 nm, wobei weitporige Aluminiumoxide tendenziell niedrigere Oberflächen besitzen. Die Poren zeigen bei derartigen weitporigen Aluminiumoxiden eine breite Verteilung von bis zu 10 nm.

Mesoporöse Oxide werden ebenfalls beispielsweise in der DE-A 44 07 326 und der DE-A 195 43 638 beschrieben. Gemäß dieser Druckschriften werden die mesoporösen Oxide dadurch hergestellt, daß man den Oxid-Vorläufern (Mono- oder Oligomere) während der Polykondensation auf dem Weg zum anorganischen Oxid ein kationisches, anionisches oder nichtionisches Tensid als strukturdirigierendes Reagenz zusetzt. Nach der eigentlichen Synthese werden gemäß dieser Druckschriften die Tenside als Strukturbildner thermisch entfernt, beispielsweise durch Brennen unter Luft bei 350 bis 600 °C, wobei ein mesoporöses, rein anorganisches Oxid erhalten wird.

"Mesoporös" bedeutet, daß der Durchmesser der Poren im Festkörper ca. 2 bis ca. 50 nm beträgt.

Die Poren der gemäß dem Stand der Technik beschriebenen mesoporösen Oxide sind überwiegend auf den Bereich von ungefähr 2 nm begrenzt.

Für einige Anwendungen, wie z. B. die Verwendung als Heterogenkatalysator, sind jedoch preiswert herzustellende, Oxide enthaltende Feststoffe mit größeren Poren wünschenswert, um bei der Umsetzung einen optimalen Stofftransport zu ermöglichen. Dies wird bei kommerziell erhältlichen Aluminiumoxiden auch teilweise erreicht, jedoch weisen diese eine breite Verteilung des Porendurchmessers auf und besitzen niedrige spezifische Oberflächen. Ferner kann es beim Tempern zur Ausbildung verschiedener kristalliner Phasen des Aluminiumoxids (beispielsweise γ-Al₂O₃ oder, bei noch höheren Temperaturen, α-Al₂O₃) kommen, die dann teilweise über inhärente katalytische Eigenschaften, wie z. B. Lewis- oder Broensted-Azidität, verfügen und damit einen unerwünschten Einfluß auf die zu katalysierende Umsetzung nehmen.

In Anbetracht des obigen Standes der Technik liegt der vorliegenden Erfindung die Aufgabe zugrunde, einen Aluminiumoxid enthaltenden Feststoff, der sich für eine Vielzahl von Anwendungen, eignet, sowie ein Verfahren zu dessen Herstellung bereitzustellen, mit dem es gelingt, den Aluminiumoxid enthaltenden Feststoff in wirtschaftlich günstiger Weise, d. h. preiswert, herzustellen.

Überraschenderweise wurde nun gefunden, daß diese Aufgabe dadurch gelöst werden kann, daß man bei der Herstellung des Aluminiumoxid enthaltenden Feststoffs als Strukturbildner ein Membranlipid verwendet.

Diese Aufgabe wird gelöst durch ein Verfahren zur Herstellung eines Aluminiumoxid enthaltenden Feststoffs, das die folgende Stufe (I) umfaßt:
(I) Inkontaktbringen mindestens eines Vorläufers von Aluminiumoxid mit mindestens einem Strukturbildner in einem flüssigen Medium,
dadurch gekennzeichnet, daß
der Strukturbildner ein Membranlipid oder ein Gemisch aus zwei oder mehr davon ist.

Fig. 1 zeigt das Ergebnis der Stickstoff-Adsorption am Feststoff gemäß Beispiel 1. Dabei bezeichnet p/p⁰ den Relativdruck, N₂^{ad} den am Feststoff adsorbierten Stickstoff, + die bei der Adsorption aufgenommenen und ++ die bei der Desorption aufgenommenen Meßwerte.

Fig. 2 zeigt das Röntgen-Diffraktogramm des Feststoffs gemäß Beispiel 1. Dabei bezeichnet I die Intensität des registrierten Signals in Counts.

Fig. 2a und 2b zeigen Ausschnittsvergrößerungen dieses Röntgen-Diffraktogramms.

Der Begriff "Strukturbildner" bezeichnet eine Substanz, die zunächst mit den Ausgangsmaterialien vermischt wird und anschließend, z. B. durch thermische Behandlung, aus dem resultierenden Feststoff entfernt wird, wobei die Bereiche, in denen sich der Strukturbildner befand, im Feststoff als Poren ausgebildet sind.

Wie bereits erwähnt, werden als Strukturbildner erfindungsgemäß Membranlipide, wie z. B. Phospholipide, Glykolipide und Cholesterin, verwendet. Dabei wird im Rahmen der vorliegenden Anmeldung der Begriff "Membranlipid" entsprechend der Definition in "L. Stryer - Biochemie", Spektrum der Wissenschaft Verlagsgesellschaft mbH, 1990, S. 296 verwendet, der relativ kleine Moleküle mit einem hydrophilen und hydrophoben Anteil bezeichnet, die in wäßrigen Medien spontan geschlossene bimolekulare Schichten ausbilden. Derartige Membranlipide werden in dem oben erwähnten Lehrbuch der Biochemie von L. Stryer auf den Seiten 296ff. ausführlich beschrieben. Darüber hinaus werden speziell Phospholipide in "Römpp-Chemielexikon", 9. Aufl. Bd. 4, S. 3383/3384, Thieme-Verlag 1991 sowie in einem Artikel von Eibl in Angew. Chem. 96 (1984), S. 247 - 262 beschrieben.

Beispiele für vorzugsweise verwendete Membranlipide sind Sphingolipide, d. h. Phospholipide, die sich von Sphingosin ableiten, Phosphoglyceride, d. h. Phospholipide, die sich von Glycerin ableiten, wie z. B. Phosphatidat, Phosphatidylserin, Phosphatidylethanolamin, Phosphatidylcholin, Phosphatidylinositol, Diphosphatidylglycerin, Glykolipide, wie z. B. Cerebrosid und Cholesterin.

Vorzugsweise werden jedoch Phospholipide als Strukturbildner verwendet.

Selbstverständlich können auch Gemische aus zwei oder mehr Membranlipiden, vorzugsweise Gemische aus zwei oder mehr der oben genannten Membranlipide, als Stukturbildner verwendet werden.

Die Menge des eingesetzten Membranlipids unterliegt im allgemeinen keinen besonderen Beschränkungen. Sie beträgt vorzugsweise ungefähr 0,05 bis ungefähr 50 Gew.-%, weiter bevorzugt ungefähr 2 bis ungefähr 10 Gew.-%, jeweils bezogen auf den Syntheseansatz, d. h. die gesamte Menge an Vorläufer von Aluminiumoxid, Strukturbildner und flüssigem Medium.

Als flüssiges Medium werden beim erfindungsgemäßen Verfahren vorzugsweise wäßrige Lösungen, wie z. B. Gemische aus Wasser und Alkoholen, wie z.B. Ethanol und/oder Isopropanol, verwendet. Das Inkontaktbringen kann jedoch auch in Wasser oder einem organischen Lösungsmittel oder einem Gemisch verschiedener organischer Lösungsmittel erfolgen.

Bevorzugt verwendet man Alkohole als Lösungsmittel, die aus der Hydrolyse der Aluminiumoxid-Vorläufer entstehen, d.h. z.B. Ethanol bei Verwendung von Aluminiumethanolat (Al(OEt)₃), i-Propanol bei Verwendung von Aluminiumisopropylat und Butanol bei Verwendung von Aluminiumbutylat.

Als Vorläufer von Aluminiumoxid können alle Verbindungen eingesetzt werden, die durch Calcinieren an der Luft bei erhöhten Temperaturen in Aluminiumoxid umgewandelt werden. Der Vorläufer kann dabei z.B. als metallorganische Komponente, wie z.B. als Alkoholat, als Grignard-Verbindung, als Alkylat, als Chelat mit z.B. Acetylacetonat als eine in einem organischen Lösungsmittel lösliche Form oder in Form löslicher Salze, als Hydroxid oder als Kolloid in wäßriger Phase, oder in einer Kombination aus zwei oder mehr davon, eingesetzt werden.

Das Inkontaktbringen kann sowohl im basischen, im sauren als auch im neutralen pH-Bereich durchgeführt werden, wobei jedoch der saure pH-Bereich, insbesondere ein pH-Wert von 1 bis 5, bevorzugt ist.

Nach dem Zusammengeben des Vorläufers von Aluminiumoxid und dem Strukturbildner in einem flüssigen Medium wird die entstehende Suspension auf den geeigneten pH-Wert gebracht und ungefähr 0,5 bis ungefähr 72, vorzugsweise ungefähr 1 bis ungefähr 48 und insbesondere ungefähr 10 bis ungefähr 30 Stunden lang bei einer Temperatur im Bereich von vorzugsweise ungefähr -10 bis ungefähr 150 °C, weiter bevorzugt ungefähr 10 bis ungefähr 90 °C und insbesondere ungefähr 20 bis ungefähr 65 °C gerührt. Der Druck während des Inkontaktbringens der oben beschriebenen Komponenten beträgt vorzugsweise ungefähr 0,4 bis ungefähr 300 bar, weiter bevorzugt ungefähr 0,8 bis 150 bar und insbesondere ungefähr 1 bis ungefähr 10 bar.

Anschließend wird die entstehende Suspension vom flüssigen Medium abgetrennt, beispielsweise durch Zentrifugation oder einfaches Abfiltrieren, und anschließend getrocknet. Bei der Trocknung wird vorzugsweise so vorgegangen, daß zunächst ungefähr 5 bis ungefähr 72 Stunden, vorzugsweise ungefähr 10 bis ungefähr 48 Stunden und insbesondere ungefähr 20 bis ungefähr 30 Stunden bei Umgebungstemperatur getrocknet und anschließend bei erhöhter Temperatur in einem Bereich von ungefähr 50 bis ungefähr 100 °C, vorzugsweise ungefähr 55 bis ungefähr 70 °C über mehrere Stunden hinweg nachgetrocknet wird.

Der so getrocknete, Aluminiumoxid enthaltende Feststoff wird anschließend bei ungefähr 350 bis ungefähr 800 °C, vorzugsweise ungefähr 400 bis ungefähr 700 °C und insbesondere bei ungefähr 450 bis ungefähr 600 °C ungefähr 2 bis ungefähr 10, vorzugsweise ungefähr 4 bis ungefähr 6 Stunden lang in Anwesenheit von Sauerstoff, vorzugsweise unter Luft, calciniert.

Während des Inkontaktbringens des Vorläufers von Aluminiumoxid mit dem Strukturbildner können noch folgende weitere Bestandteile zugesetzt werden:
- Pharmakologisch wirksame organische oder anorganische Verbindungen, wie z.B. Schmerzmittel oder Herz-Kreislaufmittel, wobei der Einbau dieser Verbindungen in den Aluminiumoxid enthaltenden Feststoff eine retardierte Freigabe der pharmakologisch wirksamen Verbindung bei der Applikation mit sich bringt;
- Enzyme für biotechnologische Anwendung, wie z.B. Oxidasen, Reduktasen, Transferasen, Hydrolasen, Lyasen, Isomerasen, Ligasen sowie semesynthetische und künstliche Enzyme, wie sie beispielsweise in "Science", 223 (1984) S. 165ff bzw. in "Cold Spring Harbor Symp. Quant Biol." 52 (1987), S. 75 - 81 und "Tetrahedron" 40 (1994), S. 269 -292 beschrieben sind, deren diesbezüglicher Inhalt vollumfänglich durch Bezugnahme in den Kontext der vorliegenden Anmeldung einbezogen wird;
- Pigmente, wie z.B. ferromagnetische Pigmente, wie z.B. Chrom(IV)dioxid, Ferrite, Eisenoxide, Eisen oder Eisenlegierungen, weitere anorganische Pigmente, wie z.B. Kreide, Graphit, Titan-, Bleioder Zinkweiß, Ruß, Leuchtpigmente, wie z.B. Zinksulfid oder Erdalkalimetallaluminate, organische Pigmente, wie z.B. Azo-Pigmente, Indigoide-, Phthalocyanin-, Metallkomplex- oder Diketopyrrolopyrrol-Pigmente, die dann ebenfalls im erhaltenen, Aluminiumoxid enthaltenden Festkörper verkapselt vorliegen.

Ferner können, insbesondere über die wäßrige Phase, bei der Herstellung des erfindungsgemäßen, Aluminiumoxid enthaltenden Feststoffs noch eine ionische Verbindung eines Elements der ersten bis dritten Hauptgruppe, außer Aluminium, oder der ersten bis achten Nebengruppe des Periodensystems, der Lanthanoiden, Silizium, Germanium, Zinn, Blei, Phosphor, Antimon, Wismut, Schwefel, Selen, Tellur oder ein Gemisch aus zwei oder mehr davon, vorzugsweise Natrium, Kalium, Calcium, Magnesium, Beryllium, Bor, Gallium, Indium, Silizium, Germanium, Zinn, Blei, Antimon, Wismut, Scandium, Yttrium, Lanthan, Titan, Zirkonium, Hafnium, Vanadium, Niob, Tantal, Chrom, Molybdän, Wolfram, Mangan, Rhenium, Eisen, Cobalt, Nickel, Ruthenium, Kupfer, Zink, Cadmium, Quecksilber, Cer, Europium, Thorium, Uran oder ein Gemisch aus zwei oder mehr davon, weiter bevorzugt Natrium, Kalium, Calcium, Magnesium, Beryllium, Bor, Gallium, Indium, Silizium, Germanium, Zinn, Blei, Wismut, Titan, Zirkonium, Hafnium, Vanadium, Niob, Tantal, Chrom, Molybdän, Wolfram, Mangan, Rhenium Eisen, Cobalt, Nickel, Ruthenium, Kupfer, Zink, Cadmium, Quecksilber, Cer oder ein Gemisch aus zwei oder mehr davon zugesetzt werden.

Die oben genannten Elemente können insbesondere in Form ihrer Sulfate, Phosphate, Nitrate, Carbonate, Halogenide und Perchlorate sowie leicht hydrolysierbaren metallorganischen Verbindungen, wie z.B. Alkoholaten, Chelaten, Carboxylaten, vorzugsweise in Form von Sulfaten, Phosphaten und Nitraten, weiter bevorzugt in Form von Sulfaten und Nitraten eingesetzt werden.

Ferner können sie auch in Form von Isopoly- oder Heteropolykationen eingesetzt werden, wie dies in den eingangs erwähnten Druckschriften DE-A 44 07 326 und der DE-A 195 43 638 beschrieben wird.

Gemäß einer weiteren Ausführungsform der vorliegenden Erfindung kann das Gemisch aus dem Vorläufer von Aluminiumoxid, dem Strukturbildner und dem flüssigen Medium auf einen inerten porösen Träger aufgebracht werden, wie z.B. ein poröses Glas, ein Kieselgel, Diatomenerde oder Tonerde, eine Keramik, ein Metall, eine Metallpackung oder auch ein Metallnetz, wie sie beispielsweise für statische Mischer oder Packungen in Kolonnen zur Reaktivdestillation eingesetzt werden. Dadurch besteht die Möglichkeit, im Rahmen des erfindungsgemäßen Verfahrens mit einem Aluminiumoxid enthaltenden Feststoff belegte oder imprägnierte inerte Träger herzustellen, wobei Verbundmaterialien entstehen, die gegenüber den Ausgangsmaterialien eine verbesserte mechanische Stabilität und verbesserte Durchströmbarkeit im Reaktor für die katalytische Umsetzung aufweisen.

Dabei sind insbesondere metallische Trägermaterialien, wie beispielsweise die Edelstähle mit Werkstoffnummern 1.4767, 1.4401, 2.4610, 1.4765, 1.4847, 1.4301 usw. zu nennen, da sie vor der Belegung/Imprägnierung mit den Feststoffen durch eine Temperung eine Aufrauhung ihrer Oberfläche erhalten können. Insbesondere bevorzugt werden als Netzmaterial Kanthal (Werkstoffnr. 1.4767) bzw. Metalle, die Aluminium enthalten, eingesetzt. Kanthal ist eine Legierung, die ungefähr 75 Gew.-% Fe, ungefähr 20 Gew.% Cr und ungefähr 5 Gew.-% Al enthält. Für die Temperung werden die oben erwähnten metallischen Träger bei Temperaturen von 600 bis 1100, vorzugsweise 800 bis 1000 °C, eine bis zwanzig, vorzugsweise eine bis zehn Stunden an der Luft erhitzt und wieder abgekühlt. Diese Vorbehandlung ist in der EP-A-0 564 830 beschrieben und wichtig, da diese Temperungsbehandlung die Verbindung der Feststoffe mit dem metallischen Träger signifikant verbessert.

Führt man das Inkontaktbringen an einer ruhenden Grenzfläche, d. h. an einer Grenzfläche zweier nichtmischbarer Flüssigkeiten, durch, so kann der Aluminiumoxid enthaltende Feststoff in Form dünner Filme oder Schichten isoliert werden, da es in Anwesenheit einer derartigen Grenzfläche möglich ist, den Feststoff bei der Herstellung in eine bestimmte gerichtete Struktur wie z.B. einen dünnen Film, zu bringen. Weitere Details sind der DE-A 196 24 862 zu entnehmen. Die so erhaltenen dünnen Filme oder Schichten können in Membran-, Trenn- und Reinigungsverfahren eingesetzt werden oder auch zur Informationsspeicherung genutzt werden. Derartige Anwendungen sind beispielsweise in der DE-A 44 24 221 beschrieben.

Derartige Festkörper können insbesondere für elektronische, optische oder elektrooptische Einsatzgebiete genutzt werden, die entsprechenden Membranen finden Verwendung in der katalytischen Umsetzung in Membranreaktoren oder bei Reaktivdestillationen. Demgemäß betrifft die vorliegende Erfindung auch die Verwendung des erfindungsgemäßen Feststoffs bzw. eines Feststoffs, der mittels des erfindungsgemäßen Verfahrens hergestellt wird, als Katalysator oder Katalysatorträger, als Matrix für Aktivkomponenten, in Membran-, Trenn- oder Reinigungsverfahren, zur Herstellung von elektrischen, optischen oder elektrooptischen Bauelementen, wie z.B. Schaltelementen oder Sensoren, zur Herstellung von Oxidkeramiken oder zur Stofftrennung, als Adsorptionsmittel, als Füllstoff, insbesondere in Polymeren, als Flammschutzmittel und als Schleif- und Poliermittel.

Sofern der erfindungsgemäß hergestellte Feststoff als Katalysator oder Katalysatorträger eingesetzt wird, findet er Verwendung in der Oxyfunktionalisierung von Kohlenwasserstoffen, der Oxidation von Olefinen zu Oxiranen, der Alkylierung von Aromaten, der Hydrierung, Dehydrierung, Hydratisierung, Dehydratisierung, Isomerisierung, einer Additionsreaktion, einer Eliminierungsreaktion, der nucleophilen und elektrophilen Substitution, Dehydrocyclisierung, Hydroxylierung von Heteroaromaten, Hydroxylierung von Aromaten, Epoxid-Aldehyd-Umlagerung, Aminierung eines monomeren oder oligomeren Olefins, einer Kondensationsreaktion vom Aldoltyp, einer Polymerisationsreaktion, Veresterung und Veretherung, der katalytischen Umsetzung von Ab- und Rauchgasen sowie zur Stickoxidentfernung.

Der erfindungsgemäße Feststoff weist insbesondere folgende Eigenschaften auf:
- Er zeigt in der röntgenographischen Analyse die intensivsten Reflexe im Winkelbereich kleiner 4° (2 θ), bei der Bestimmung mit Cu-K_{α}-Strahlung.
- Der Feststoff weist Poren im Bereich von ungefähr 2 bis ungefähr 50 nm, vorzugsweise ungefähr 4 bis ungefähr 30 nm und insbesondere ungefähr 6 bis ungefähr 10 nm auf, wobei der Porendurchmesser mittels Stickstoffadsorption bei 77 K ermittelt wird. Die bei gleichen Bedingungen gemessene spezifische Oberfläche nach Barret-Joyner Halenda-Auswertung gemäß J. Am. Chem. Soc., 73 (1951), S. 373-380 liegt oberhalb von ungefähr 100 m²/g, vorzugsweise oberhalb 250 m²/g und insbesondere bei ungefähr 300 bis 550 m²/g.
- Das ebenfalls mittels Stickstoffadsorption bei 77 K ermittelte Porenvolumen liegt oberhalb von ungefähr 0,2 ml/g, vorzugsweise oberhalb von ungefähr 0,25 ml/g und insbesondere bei ungefähr 0,5 ml/g bis 1,0 ml/g.

Ferner betrifft die vorliegende Erfindung die Verwendung eines Membranlipids oder eines Gemischs aus zwei oder mehr davon als Strukturbildner bei der Herstellung eines Aluminiumoxid enthaltenden Feststoffs.

Die nachfolgenden Beispiele sollen Herstellung und Eigenschaften des erfindungsgemäßen Feststoffs näher erläutern und in Bezug setzen zu einem Vergleichsbeispiel, das gemäß einem Verfahren des Standes der Technik, d. h. ohne Zusatz eines Membranlipids hergestellt wurde.

### BEISPIELE

### Beispiel 1

In einem 2 1 Vierhalskolben wurde eine Lösung aus 205,8 g Aluminiumtriisopropylat (Fa. Merck), 300 g Ethanol und 81,0 g Isopropanol hergestellt und 30 min homogenisiert. Danach gab man 50,0 g Sojalecithin PC 4045 mit 40 - 45 % Phosphatidylcholin, 10% Phosphatidylethanolamin, 2% Phosphatidylinositol (Fa. G. Gienow, Pinneberg) zu. Anschließend wurde eine Lösung aus 650,0 g deionisiertem Wasser und 7,5 g einer Salzsäurelösung (10 Gew.-%) zugegeben, wobei sich eine Suspension bildete. Die Suspension wurde 24 h lang bei Raumtemperatur gerührt, abfiltriert und 24 h lang an Luft getrocknet. Danach wurde über Nacht bei 60 °C nachgetrocknet. Die Auswaage betrug 124 g Feststoff. Abschließend wurde bei 500 °C 5 Stunden lang unter Luft calciniert. Der Calcinierungsverlust betrug 63 Gew.-% auf den eingesetzten Feststoff.

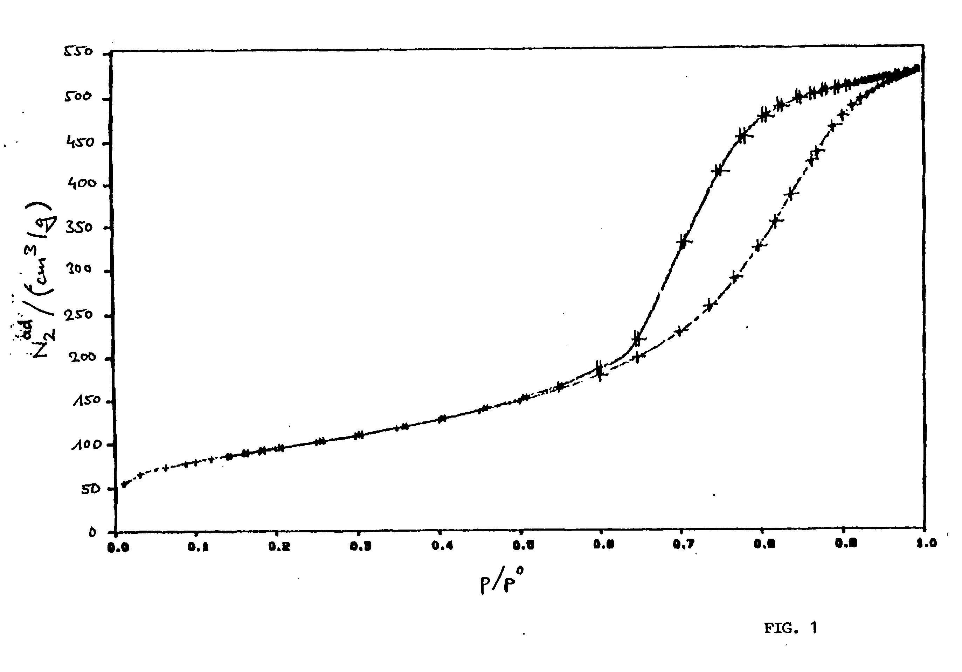

Mittels Stickstoffadsorption bei 77 K fand man die in Fig. 1 gezeigte typische Hysterese im Relativdruckbereich von p/p⁰ größer 0,6. Unter Verwendung des BJH-Modells ergab sich daraus für Poren im Bereich von 6 bis 7 nm eine Porenoberfläche von 497 m²/g. Das entsprechende Porenvolumen betrug 0,81 ml/g, gemessen bei einem Relativdruck von p/p⁰ = 0,98. Der häufigste Porendurchmesser betrug ca. 7 nm.

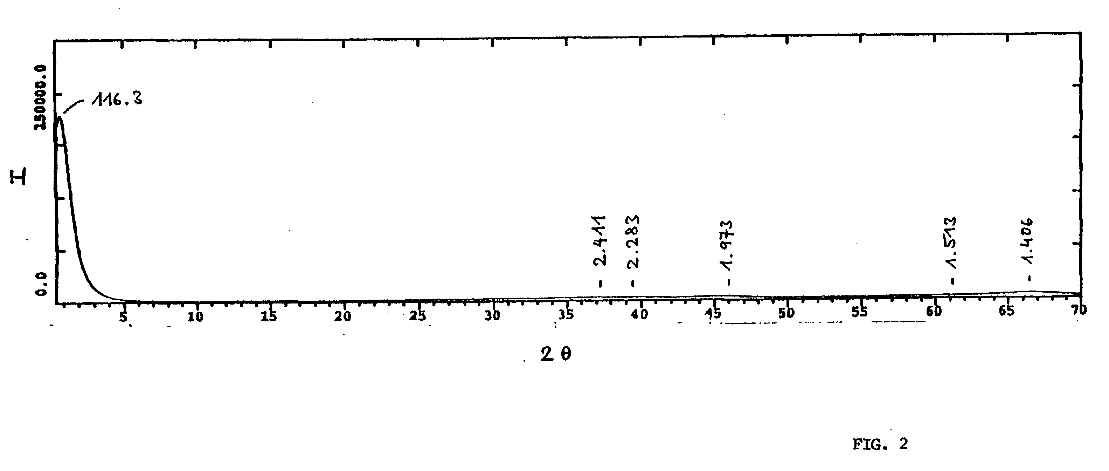

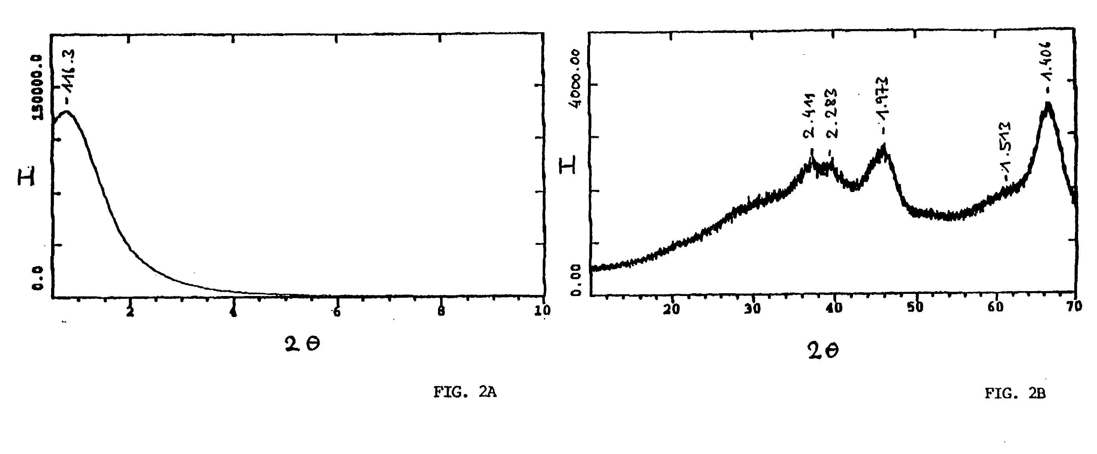

Röntgenographisch zeigte der so erhaltene Feststoff die höchsten Reflex-Intensitäten bei Winkelwerten kleiner 4° (2 θ) (Cu-K_{α}-Strahlung). Das Diffraktogramm ist in Fig. 2, 2a, 2b wiedergegeben.

### Vergleichsbeispiel 1

In einem 2 l Vierhalskolben wurde eine Lösung aus 205,8 g Aluminiumtriisopropylat (Fa. Merck), 300 g Ethanol und 51,0 g Isopropanol hergestellt und für 30 min homogenisiert. Danach gab man eine Lösung aus 650,0 g Wasser und 7,5 g der Salzsäurelösung (10 Gew.-%) zu, wobei sich eine weiße Suspension bildete. Die weiße Suspension wurde 24 h lang bei Raumtemperatur gerührt, abfiltriert und 24 h lang an Luft bei Raumtemperatur und danach bei 60 °C über Nacht getrocknet. Die Auswaage betrug 87,7 g. Der Feststoff wurde anschließend bei 500 °C 5 Stunden lang calciniert. Der Calcinierungsverlust betrug 28,2 Gew.-%.

Röntgenographisch zeigte der so erhaltene Feststoff keinen ausgeprägten Röntgenreflex bei Winkelwerten kleiner 4° (2 θ). Es handelte sich um reines γ-Al₂O₃.

Mittels Stickstoffadsorption bei 77 K fand man typische Hysteresen im Relativdruckbereich von p/p⁰ größer 0,4. Unter Verwendung des BJH-Modells ergaben sich daraus Poren im Bereich von 2 bis 5 nm. Die Porenoberfläche nach BET betrug 289 m²/g. Das entsprechende Porenvolumen betrug lediglich 0,37 ml/g, gemessen bei einem Relativdruck von p/p⁰ von 0,98. Der häufigste Porendurchmesser betrug ca. 3,8 nm.

## Patentansprüche

1. Verfahren zur Herstellung eines Aluminiumoxid enthaltenden Feststoffs, das die folgende Stufe (I) umfaßt:
(I) Inkontaktbringen mindestens eines Vorläufers von Aluminiumoxid mit mindestens einem Strukturbildner in einem flüssigen Medium,
**dadurch gekennzeichnet, daß**
der Strukturbildner ein Membranlipid oder ein Gemisch aus zwei oder mehr davon ist.

2. Verfahren nach Anspruch 1, **dadurch gekennzeichnet, daß** das Membranlipid ein Phospholipid oder ein Gemisch aus zwei oder mehr davon ist.

3. Verfahren nach Anspruch 1 oder 2, **dadurch gekennzeichnet, daß** der Aluminiumoxid enthaltende Feststoff mindestens ein Element ausgewählt aus der Gruppe bestehend aus Natrium, Kalium, Calcium, Magnesium, Beryllium, Bor, Gallium, Indium, Silizium, Germanium, Zinn, Blei, Wismut, Titan, Zirkonium, Hafnium, Vanadium, Niob, Tantal, Chrom, Molybdän, Wolfram, Mangan, Rhenium, Eisen, Cobalt, Nickel, Ruthenium, Kupfer, Zink, Cadmium, Quecksilber und Cer umfaßt.

4. Verfahren nach einem der Ansprüche 1 bis 3, **dadurch gekennzeichnet, daß** der Vorläufer zusätzlich mit einer pharmakologisch wirksamen organischen oder anorganischen Verbindung, einem Enzym, einem Pigment oder einem Gemisch aus zwei oder mehr davon in Kontakt gebracht wird.

5. Verfahren nach einem der Ansprüche 1 bis 4, **dadurch gekennzeichnet, daß** das Inkontaktbringen in Gegenwart eines inerten porösen Trägers oder an einer ruhenden Grenzfläche durchgeführt wird.

6. Verwendung eines mittels eines Verfahrens gemäß einem der Ansprüche 1 bis 5 hergestellten Feststoffs als als Matrix für Aktivkomponenten, in Membran-, Trenn- oder Reinigungsverfahren, zur Herstellung von elektrischen, optischen oder elektrooptischen Bauelementen, zur Herstellung von Oxidkeramiken oder zur Stofftrennung, als Füllstoff und als Flammschutzmittel und als Schleif- und Poliermittel.

7. Verwendung eines mittels eines verfahrens gemäß einem der Ansprüche 1 bis 5 hergestellten Feststoffs als Katalysator oder Katalysatorträger bei der Oxyfunktionalisierung von Kohlenwasserstoffen, der Oxidation von Olefinen zu Oxiranen, der Alkylierung von Aromaten, der Hydrierung, Dehydrierung, Hydratisierung, Dehydratisierung, Isomerisierung, einer Additionsreaktion, einer Eliminierungsreaktion, einer nucleophilen und elektrophilen Substitution, einer Dehydrocyclisierung, Hydroxylierung von Heteroaromaten, Hydroxylierung von Aromaten, Epoxid-Aldehyd-Umlagerung, Aminierung eines monomeren oder oligomeren Olefins, einer Kondensationsreaktion vom Aldoltyp, einer Polymerisationsreaktion, einer Veresterung und Veretherung, der katalytischen Umsetzung von Ab- und Rauchgasen sowie zur Stickoxidentfernung.

8. Verwendung eines Membranlipids ober eines Gemisches aus zwei ober mehr davon als Strukturbildner bei der Herstellung eines Aluminiumoxid enthaltenden Feststoffs.

## Claims

1. A process for preparing an alumina solid, which comprises the step of contacting in a liquid medium at least one alumina precursor with at least one template comprising a membrane lipid or a mixture of two or more thereof.

2. A process as claimed in claim 1, wherein the membrane lipid is a phospholipid or a mixture of two or more thereof.

3. A process as claimed in claim 1 or 2, wherein the alumina solid comprises at least one element selected from the group consisting of sodium, potassium, calcium, magnesium, beryllium, boron, gallium, indium, silicon, germanium, tin, lead, bismuth, titanium, zirconium, hafnium, vanadium, niobium, tantalum, chromium, molybdenum, tungsten, manganese, rhenium, iron, cobalt, nickel, ruthenium, copper, zinc, cadmium, mercury and cerium.

4. A process as claimed in any of claims 1 to 3, wherein the precursor is additionally contacted with a pharmacologically active organic or inorganic compound, an enzyme, a pigment or a mixture of two or more thereof.

5. A process as claimed in any of claims 1 to 4, wherein the contacting is carried out in the presence of an inert porous support or at a static interface.

6. The use of a solid prepared by a process as claimed in any of claims 1 to 5 as matrix for active components, in membrane, separation or purification processes, for producing electrical, optical or electro-optic components, for producing oxide ceramics or for separating substances, as filler, as fire retardant and as abrasives and polishing materials.

7. A use of a solid prepared by a process as claimed in any of claims 1 to 5 as a catalyst or catalyst support in hydrocarbon oxyfunctionalization, olefin oxidation to give oxiranes, aromatics alkylation, hydrogenation, dehydrogenation, hydration, dehydration, isomerization, an addition reaction, an elimination reaction, a nucleophilic or electrophilic substitution, a dehydrocyclization, hydroxylation of heteroatoms, aromatics hydroxylation, epoxy-aldehyde rearrangement, amination of monomeric or oligomeric olefins, an aldol type condensation reaction, a polymerization reaction, an esterification or etherification, the catalytic conversion of exhaust gases and flue gases or for nitrogen oxide removal.

8. The use of a membrane lipid or a mixture of two or more thereof as templates in the preparation of an alumina solid.

## Revendications

1. Procédé de fabrication d'un solide contenant de l'oxyde d'aluminium, qui comprend l'étape (I) suivante :
(I) Mise en contact d'au moins un précurseur d'oxyde d'aluminium avec au moins un formateur de structure dans un milieu liquide, **caractérisé en ce que** le formateur de structure est un lipide membranaire ou un mélange de deux ou plus de celui-ci.

2. Procédé selon la revendication 1, **caractérisé en ce que** le lipide membranaire est un phospholipide ou un mélange de deux ou plus de celui-ci.

3. Procédé selon la revendication 1 ou 2, **caractérisé en ce que** le solide contenant de l'oxyde d'aluminium comprend au moins un élément choisi dans le groupe formé par le sodium, le potassium, le calcium, le magnésium, le béryllium, le bore, le gallium, l'indium, le silicium, le germanium, l'étain, le plomb, le bismuth, le titane, le zirconium, l'hafnium, le vanadium, le niobium, le tantale, le chrome, le molybdène, le tungstène, le manganèse, le rhénium, le fer, le cobalt, le nickel, le ruthénium, le cuivre, le zinc, le cadmium, le mercure et le cérium.

4. Procédé selon l'une quelconque des revendications 1 à 3, **caractérisé en ce que** le précurseur est de plus mis en contact avec une liaison organique ou anorganique efficace pharmacologiquement, avec un enzyme, un pigment ou un mélange de deux ou plus de ceux-ci.

5. Procédé selon l'une quelconque des revendications 1 à 4, **caractérisé en ce que** la mise en contact est effectuée en présence d'un support inerte poreux ou sur une interface au repos.

6. Utilisation d'un solide fabriqué au moyen d'un procédé selon l'une quelconque des revendications 1 à 5 en tant que matrice pour des composants actifs, dans des procédés membranaires, de séparation ou de purification, pour la fabrication de composants électriques, optiques ou électro-optiques, pour la fabrication de céramiques d'oxyde ou pour la séparation de substances comme charges et comme ignifugeants et comme abrasifs et substances à polir.

7. Utilisation d'un solide fabriqué au moyen d'un procédé selon l'une quelconque des revendications 1 à 5 en tant que catalyseur ou support de catalyseur lors de la fonctionnalisation oxydante d'hydrocabures, de l'oxydation d'oléfines en oxirannes, de l'alkylation d'aromates, de l'hydrogénation, déshydrogénation, hydratation, déshydratation, isomérisation, d'une réaction d'addition, d'une réaction d'élimination, d'une substitution nucléophile et électrophile, d'une déhydrocyclisation, hydroxylation d'hétéroaromates, hydroxylation d'aromates, réarrangement aldéhyde ou époxyde, amination d'une oléfine monomère ou oligomère, d'une réaction de condensation du type aldol, d'une réaction de polymérisation, d'une ésterification et étherification, de la réaction catalytique de gaz résiduels et de gaz de fumée ainsi que l'élimination d'oxyde d'azote.

8. Utilisation d'un lipide membranaire ou d'un mélange de deux ou plus de ceux-ci en tant que formateur de structure lors de la fabrication d'un solide contenant un oxyde d'aluminium
